# EUROPEAN PATENT SPECIFICATION

(11) **EP 1 968 552 B1**
(45) Date of publication and mention of the grant of the patent: **22.07.2015**
(21) Application number: 06831450.9
(22) Date of filing: 22.12.2006
(51) Int. Cl.: A61K 31/5415

(54) **blister pack**
Blisterpackung
EMBALLAGE-COQUE

(30) Priority: 04.01.2006 GB 0600075
(43) Date of publication of application: 17.09.2008
(73) Proprietor: Alliance Pharmaceuticals Limited, Chippenham Wiltshire SN15 2BB (GB)
(72) Inventor: CAIRNS, Graham, Hull HU8 7DS (GB); HYDE, Neil, Hull HU8 7DS (GB); KNOWLES, Andrew, Hull HU8 7DS (GB); MILLER, Kerry, Anne, Hull HU8 7DS (GB)
(74) Representative: Bailey, Sam Rogerson
(86) International application number: PCT/GB2006/004891
(87) International publication number: WO 2007/077421

(56) References cited:
- EP-A- 0 132 145
- EP-A2- 0 180 364
- WO-A-01/04019
- US-A1- 2005 061 705
- ANONYMOUS: "Buccastem" INTERNET ARTICLE, REVISED APRIL 2001, XP002425956 Retrieved from the Internet: URL:http://emc.medicines.org.uk/emc/assets /c/html/DisplayDoc.asp?DocumentID=18293>
- ANONYMOUS: "MITIL Tablets" INTERNET ARTICLE, FEB. 2004, XP002425957 Retrieved from the Internet: URL:http://home.intekom.com/pharm/lennon/m itil.html>
- ANONYMOUS: "Securitainers" INTERNET ARTICLE, [Online] 2003, XP002429392 Retrieved from the Internet: URL:http://www.jjpack.com/personal_care_ph armaceuticals_products_detail.php?productI D=162> [retrieved on 2007-03-21]

## Description

This invention relates to a pack of medicinal tablets, in particular to a pack of medicinal tablets containing prochlorperazine maleate, as active ingredient.

An existing product containing prochlorperazine maleate is BUCCASTEM (trade mark), a product sold to combat nausea, vomiting, vertigo and migraine.

We have discovered unexpectedly that in a traditional PVC/PVdC blister pack having tablets containing prochlorperazine maleate as the active ingredient, the stored tablets may be affected by moisture.

PVC/PVdC is a laminate of polyvinyl chloride polyvinylidene chloride. It is generally accepted to be a good oxygen and moisture barrier, suitable for the packaging of many medicinal products.

As a consequence of our finding, however, we believe it is desirable to use a better barrier material for tablets of prochlorperazine maleate.

The internet article "Buccastem" from the electronic Medicines Compendium (http://emc.medicines.org.uk) describes 3 mg prochlorperazine maleate buccal tablets. These tablets are held in 250 micron PVC aluminium foil blister packs or plastic tubs.

US 2005/0061705 describes a pharmaceutical blister pack with reduced permeability to water vapour and gas. It describes conventional blisters in which the base and/or cover foil are coated with an additional functional layer containing silicon oxide and carbon, to protect the tablets.

The present invention provides a blister pack containing medicinal tablets, formed of a metallic material, comprising a base part containing pockets for the tablets formed of a cold-formed foil comprising a metallic layer laminated to a layer of PVC, the PVC layer being the product contact surface, over which a breachable-by-tablet metallic foil is laid, wherein the tablets comprise from 3 to 10 mg of prochlorperazine maleate, and wherein the tablets in the pack are protected from damage by water.

The phenothiazine derivative in the pack of the present invention is prochlorperazine maleate.

Each tablet comprises from 3 to 10 mg of the phenothiazine derivative.

In especially preferred embodiments each tablet contains up to 8 mg of phenothiazine derivative, especially up to 6 mg.

More preferably the tablets contain 3 mg to 6 mg of phenothiazine derivative, most preferably 3 mg or, especially, 6 mg.

Preferably the tablets comprise at least one monosaccharide or disaccharide or a mixture thereof. Preferably the tablets comprise a mixture of xanthan gum and locust bean gum, preferably in a weight ratio of 3:1 to 1:1.

Preferably the weight total of the mono- and/or disaccharides relative to the combined weight of the xanthan and locust bean gums (when all of these components are present) is in the ratio of 20:1 to 5:1, more preferably 16:1 to 7.5:1.

When a monosaccharide is present it is preferably selected from glucose, galactose, fructose, mannose, mannitol and sorbitol.

When a disaccharide is present it is preferably selected from maltose, lactose and sucrose.

According to the present invention, the pack is a blister, pack.

A pack of this invention is a flexible flat pack, of a water-occlusive material. The pack is a blister pack and has a water-occlusive wall or barrier member and a water-occlusive closure, lid or other type of seal. By water-occlusive material, we mean a material which preferably has a higher degree of water occlusivity under test conditions, 40ºC and 75% Relative Humidity (RH), than a traditional PVC/PVdC material.

A blister pack of the invention comprises or is formed of a metallic material, for example aluminium or an aluminium alloy. According to the present invention the base part, containing the pockets for tablets, is of a cold-formable foil, comprising a metallic layer, over which a breachable-by-tablet metallic foil is laid, to close the pack. The cold-formable foil comprises a layer of a metallic material, for example aluminium our aluminium alloy, laminated to a layer of PVC. The layer of a metallic material may also be laminated to a layer of aliphatic polyamide. Especially preferred is a foil having a layer of aliphatic polyamide, 10-40 µm thick, then a metallic layer, 30-60 µm thick, then a layer of PVC, 40-80 µm thick, the latter being the product contact surface.

Preferably a blister pack of the invention contains at least 6 tablets, more preferably at least 8 tablets.

Preferably a blister pack of the invention contains up to 14 tablets, especially up to 12 tablets.

Preferably the pack of the invention has a use-by date at least 6 months after its date of packing; more preferably at least 9 months. The use-by date may be recorded on the pack itself or on secondary packaging therefor.

The present invention also provides a blister pack containing medicinal tablets, according to the present invention, for use in a method of providing and/or administering tablets of prochlorperazine maleate.

The invention will now be further described, by way of example, with reference to the following example.

Medicament tablets were made, containing the following ingredients.

| **Component** | **Quantity/tablet (mg)** | **Function** |
|---|---|---|
| Prochlorperazine maleate | 6.00 | Active ingredient |
| Compressible sugar | 46.32 | Filler/bulking agent |
| Povidone K30 | 3.00 | Binder |
| Locust bean gum | 1.50 | Galling agent |
| Xanthan gum | 1.50 | Gelling agent |
| Talc, sterilised | 1.00 | Glidant (compression aid) |
| Magnesium stearate | 0.50 | Lubricant (compression aid) |
| B-carotene 1% CWS | 0.18 | Colouring agent |
| TOTAL | 60.00 | |

The tablets were prepared as follows.

The solid ingredients, excluding magnesium stearate and talc, were granulated in a mixed alkanol/water solvent system. To this end, the active ingredient was suspended in alkanol, and the colourant was dissolved in water. These two components were mixed together to form a uniform alkanol/water suspension which was added to the dry powder mixture during the granulation process. The granules were dried in a tray dryer at moderate elevated temperature for approximately two hours, were sieved, and were then blended with the magnesium stearate and talc, using a tumble blender. The resulting pre-tabletting mix was then compressed on a rotary tablet press to produce round, biconvex tablets, 5.6 mm in diameter, of tablet weight 60 mg.

The resulting tablets were packed into two types of blister pack: one in accordance with the present invention, which had a cold form aluminium foil tray, heat sealed by an aluminium breachable foil lid; the other a "250/60" PVC/PVdC tray (250 µm thickness PVC, 60 µm thickness PVdC), with an aluminium breachable foil lid. The blister packs contained ten tablets. Packing was carried out in dry air. Each tablet pocket was individually sealed, by the breachable aluminium foil lid. Tablets were packed into the blister trays and sealed immediately after their manufacture.

The cold-form aluminium foil tray was a laminate of 25 µm thickness aliphatic polyamide, 46 µm thickness aluminium and 60 µm thickness PVC (the material in contact with the tablets).

The aluminium breachable foil lid was a 50 g/m² lidding foil of 12 µm PET/25 µm aluminium/6-8 g/m² PVC/PVA lacquer (product contact surface).

The tablets were then stored under constant conditions of 40ºC temperature, 75% Relative Humidity (RH). Samples were studied after six, twelve, twenty-six and thirty-nine weeks under these conditions, for visual appearance, water content and perchlorperazine maleate (PCP) content. The results are presented below:

| Time | Visual appearance PVC/PVdC | Visual appearance Alu/Alu | Water content (% w/w) PVC/PVdC | Water content (% w/w) Alu/Alu | PCP content (mg) PVC/PVdC | PCP content (mg) Alu/Alu |
|---|---|---|---|---|---|---|
| Initial | Pale yellow | Pale Yellow | 1.46 | 1.04 | 5.98 | 5.99 |
| 6 weeks | As initial | As initial | 2.04 | 1.07 | 5.93 | 5.90 |
| 12 weeks | Slightly darker | As initial | 1.55 | 0.97 | 5.93 | 5.95 |
| 26 weeks | Darker than initial, mottled, damp-looking | Very slightly paler than initial | 2.17 | 1.11 | 6.15 | 5.93 |
| 39 weeks | Darker than initial, mottled, damp-looking | Very slightly paler than initial | 3.26 | 1.12 | 5.87 | 5.90 |

Although the initial water content of the two sets of tablets was different they were from the same batch. Evidently the initial pick-up of water by the two sets of tablets differed. Whilst that in itself is of interest, of particular interest was the fact that the rate at which the two sets picked up water over an extended period. In relation to this aspect, the results showed a progressively increasing difference in their water content over time. Water-absorption was clearly shown in the tablets of the PVC/PVdC pack, but not in the tablets of the Alu/Alu pack.

Neither set of tablets showed any evidence of chemical decomposition of the active ingredient, after any of these time periods. However in the case of the PVC/PVdC pack some darkening and mottling of the tablets was observed, starting at 12 weeks. With the tablets packed into the aluminium blister pack only a very slight colour change, a slight lightening was observed, starting at 26 weeks.

## Claims

1. A blister pack, containing medicinal tablets, formed of a metallic material, comprising a base part containing pockets for the tablets formed of a cold-formed foil comprising a metallic layer laminated to a layer of PVC, the PVC layer being the product contact surface, over which a breachable-by-tablet metallic foil is laid, wherein the tablets comprise from 3 to 10 mg of prochlorperazine maleate, and wherein the tablets in the pack are protected from damage by water.

2. A pack according to claim 1, wherein the base part is formed from a cold-formed foil comprising a 10-40µm thick layer of aliphatic polyamide laminated to a 30-60µm thick metallic layer laminated to a 40-80µm thick PVC layer, the PVC layer being the product contact surface.

3. A pack as claimed in claim 1 or 2 wherein the tablets include a monosaccharide or a disaccharide or a mixture thereof.

4. A pack as claimed in claim 3 wherein the tablets comprise sucrose.

5. A pack as claimed in any preceding claim wherein the tablets comprise a mixture of xanthan gum and locust bean gum.

6. A pack as claimed in any preceding claim wherein each tablet thereof contains from 3mg to 6mg of prochlorperazine maleate.

7. A pack as claimed in any preceding claim wherein the tablets has a use-by date at least 6 months after packing into the water-occlusive pack.

8. A blister pack, containing medicinal tablets, according to any one of the preceding claims, for use in providing and/or administering tablets of prochlorperazine maleate.

## Patentansprüche

1. Medizinische Tabletten enthaltende Blisterpackung, die aus einem Metallmaterial gebildet ist, das einen Basisteil mit Taschen für die Tabletten umfasst, der aus einer kaltgeformten Folie geformt ist, die eine Metallschicht umfasst, die auf eine PVC-Schicht auflaminiert ist, wobei die PVC-Schicht die Produktkontaktoberfläche ist und mit einer von der Tablette durchbrechbaren Metallfolie überzogen ist, worin die Tabletten 3 bis 10 mg Prochlorperazinmaleat umfassen und worin die Tabletten in der Packung vor Wasserschäden geschützt sind.

2. Packung nach Anspruch 1, worin der Basisteil aus einer kaltgeformten Folie geformt ist, umfassend eine 10 bis 40 µm dicke Schicht aus aliphatischem Polyamid, das auf eine 30 bis 60 µm dicke Metallschicht auflaminiert ist, die auf eine 40 bis 80 µm dicke PVC-Schicht auflaminiert ist, wobei die PVC-Schicht die Produktkontaktoberfläche ist.

3. Packung nach Anspruch 1 oder 2, worin die Tabletten ein Monosaccharid oder ein Disaccharid oder ein Gemisch davon umfassen.

4. Packung nach Anspruch 3, worin die Tabletten Saccharose umfassen.

5. Packung nach einem der vorangegangenen Ansprüche, worin die Tabletten ein Gemisch aus Xanthangummi und Johannisbrotkernmehl umfassen.

6. Packung nach einem der vorangegangenen Ansprüche, worin jede Tablette davon 3 mg bis 6 mg Prochlorperazinmaleat enthält.

7. Packung nach einem der vorangegangenen Ansprüche, worin die Tabletten eine Aufbrauchfrist von mindestens 6 Monaten, nachdem sie in die wasserdichte Packung abgepackt worden sind, aufweisen.

8. Medizinische Tabletten enthaltende Blisterpackung nach einem der vorangegangenen Ansprüche zur Verwendung zum Bereitstellen und/oder Verabreichen von Tabletten aus Prochlorperazinmaleat.

## Revendications

1. Emballage-coque, contenant des comprimés médicinaux, formé d'un matériau métallique, comprenant une partie de base contenant des poches pour les comprimés formée d'une feuille d'aluminium formée à froid comprenant une couche métallique stratifiée sur une couche de PVC, la couche de PVC étant la surface de contact avec le produit, sur laquelle est disposée une feuille métallique pouvant être percée par le comprimé, dans lequel les comprimés comprennent de 3 à 10 mg de maléate de prochlorpérazine et dans lequel les comprimés dans l'emballage sont protégés contre un endommagement par l'eau.

2. Emballage selon la revendication 1, dans lequel la partie de base est formée à partir d'une feuille formée à froid comprenant une couche de 10 à 40 µm d'épaisseur de polyamide aliphatique stratifiée sur une couche métallique de 30 à 60 µm d'épaisseur stratifiée sur une couche de PVC de 40 à 80 µm d'épaisseur, la couche de PVC étant la surface en contact avec le produit.

3. Emballage selon la revendication 1 ou 2, dans lequel les comprimés incluent un monosaccharide ou un disaccharide ou un mélange de ceux-ci.

4. Emballage selon la revendication 3, dans lequel les comprimés comprennent du saccharose.

5. Emballage selon l'une quelconque des revendications précédentes, dans lequel les comprimés comprennent un mélange de gomme de xanthane et de gomme de caroube.

6. Emballage selon l'une quelconque des revendications précédentes, dans lequel chaque comprimé contient de 3 mg à 6 mg de maléate de prochlorpérazine.

7. Emballage selon l'une quelconque des revendications précédentes, dans lequel les comprimés ont une date de péremption d'au moins 6 mois après conditionnement dans l'emballage étanche à l'eau.

8. Emballage-coque, contenant des comprimés médicinaux, selon l'une quelconque des revendications précédentes, destiné à être utilisé pour la fourniture et/ou l'administration de comprimés de maléate de prochlorpérazine.
